# EUROPEAN PATENT SPECIFICATION

(11) **EP 2 586 523 B1**
(45) Date of publication and mention of the grant of the patent: **11.11.2020**
(21) Application number: 11797980.7
(22) Date of filing: 07.06.2011
(51) Int. Cl.: B01F 17/52, A61K 9/107, A61K 47/36, A61K 47/30

(54) **PROCESS FOR PRODUCING EMULSIFIER-PRODUCING MATERIAL, PROCESS FOR PRODUCING EMULSIFIER, EMULSIFIER FOR ORALLY ADMINISTERED COMPOSITION, AND ORALLY ADMINISTERED COMPOSITION**
VERFAHREN ZUR HERSTELLUNG EINES EMULGATORPRODUKTIONSMATERIALS, VERFAHREN ZUR HERSTELLUNG EINES EMULGATORS, EMULGATOR FÜR EINE ORAL VERABREICHTE ZUSAMMENSETZUNG UND ORAL VERABREICHTE ZUSAMMENSETZUNG
PROCÉDÉ DE PRODUCTION D'UNE MATIÈRE PRODUISANT UN ÉMULSIFIANT, PROCÉDÉ DE PRODUCTION D'UN ÉMULSIFIANT, ÉMULSIFIANT POUR COMPOSITION À ADMINISTRATION ORALE ET COMPOSITION À ADMINISTRATION ORALE

(30) Priority: 23.06.2010 JP 2010142974
(43) Date of publication of application: 01.05.2013
(73) Proprietor: Kanagawa University, Yokohama-shi, Kanagawa 221-8686 (JP)
(72) Inventor: TAJIMA Kazuo, Yokohama-shi Kanagawa 221-8686 (JP); IMAI Yoko, Yokohama-shi Kanagawa 221-8686 (JP)
(74) Representative: Ström & Gulliksson AB
(86) International application number: PCT/JP2011/063016
(87) International publication number: WO 2011/162094

(56) References cited:
- WO-A2-2005/096711
- CA-A1- 2 050 599
- JP-A- 62 003 748
- JP-A- 2005 194 419
- JP-A- 2008 093 657
- US-A- 3 184 385
- US-A- 5 676 994
- US-A1- 2007 261 293

## Description

### TECHNICAL FIELD

The present invention relates to a process for producing an emulsifier.

### BACKGROUND ART

Hitherto, when emulsifying or dispersing functional oily bases or functional granules into water, surfactants are selected depending on the required HLB of the functional oily bases or the nature of the surface of the granules, to realize the emulsification or dispersion. In addition, the required HLB value of the surfactants that are employed as an emulsifier needs to be properly chosen in accordance with the respective formations of O/W emulsions and W/O emulsions. Further, because of insufficient thermal stability and aging stability of the resultant emulsions, a mixture of a wide variety of surfactants are used (see, for example, Non-Patent Documents 1 to 4).

However, the surfactants pose serious problems such as environmental pollution, since they have low biodegradability and cause foaming. Furthermore, although physicochemical emulsification techniques such as an HLB technique, a phase-inversion emulsification technique, a phase-inversion temperature emulsification technique, and a gel emulsification technique are commonly employed as a preparation method of emulsified formulations of the functional oily bases, all the above-described techniques are based, for the preparation of the emulsions, on the effect of reducing interface energy of an oil/water interface and thermodynamically stabilizing the system. Therefore, these techniques require extremely cumbersome and tremendous efforts to select the optimal emulsifier; furthermore, if many kinds of oils are present in admixture, stable emulsification by means of the above techniques is nearly impossible.

Thereupon, Patent Document 1 discloses an emulsifier that contains hydrophilic nanoparticles separated by dispersing a biopolymer into water and treating it with heat or urea.

Thereupon, Patent Document 2 discloses a stable and non-separable composition comprised of starch and oil that can be prepared in the absence of external emulsifying or dispersing agents by thoroughly solubilizing an aqueous dispersion of the starch at elevated temperatures and incorporating the oil into the non-retrograded starch under conditions of high turbulence.

Thereupon, Patent Document 3 discloses an emulsification technique which permits the formation of functional oil/water or functional granules/water emulsion systems excellent in thermal stability and long-term stability and which can attain the emulsification independent of required HLB of the functional oils, and the dispersion independent of surface properties of the functional granules.

Thereupon, Patent Document 4 discloses dry, fat-soluble vitamin-active products in free-flowing, finely divided form.

Thereupon, Patent Document 5 discloses encapsulating or coating carrier substance used for the encapsulation or coating of one or more active compounds in a or with a carrier substance is a mixture of essentially native starch and at least one agent at least partially swelling the starch and is mixed, for encapsulation or for coating, with the active compound and at least one emulsifier.
Patent Document 1: Japanese Patent Publication No. 3855203
Patent Document 2: US Patent Publication No. US5676994A
Patent Document 3: US Patent Publication No. US2007/261293A1
Patent Document 4: US Patent Publication No. US3184385A
Patent Document 5: CA Patent Publication No. CA2050599A1
[Non-Patent Document 1] "Emulsion Science" Edited by P. Sherman, Academic Press Inc. (1969)
[Non-Patent Document 2] "Microemulsions-Theory and Practice" Edited by Leon M. Price, Academic Press Inc. (1977)
[Non-Patent Document 3] "Nyuka-Kayouka no Gijutsu (Technique of Emulsification and Solubilization)" edited by Susumu Tsuji, Kougakutosho Ltd. (1976)
[Non-Patent Document 4] "Kinousei Kaimenkasseizai no Kaihatsu Gijutsu (Developing Technique of Functional Surfactants)", CMC Publishing Co., Ltd. (1998)

### DISCLOSURE OF THE INVENTION

### Problems to be Solved by the Invention

However, in the method disclosed in Patent Document 1, emulsifying function of the emulsifier is not fully exhibited due to large variation in particle diameter among the resultant hydrophilic nanoparticles, or homogeneity of the emulsifier is insufficient. In this case, if mechanical forces such as agitation are unduly applied for the purpose of inhibiting the variation in particle diameter among the hydrophilic nanoparticles, the biopolymer may be denatured and the emulsifying functions may be impaired. Demands for solving the problems with respect to the emulsifiers that contain the hydrophilic nanoparticles are especially high in the emulsification of orally administered objects (for example, beverage and food compositions).

The present invention was developed in view of the above mentioned situation. An object of the present invention is to provide a process for producing an emulsifier-producing material that is capable of inhibiting the variation in particle diameter among the hydrophilic nanoparticles and the impairment of the emulsifying functions, and a process for producing an emulsifier. In addition, another object of the present invention is to provide an emulsifier for an orally administered composition that is produced by the process according to the present invention and is superior in safety, and an orally administered composition containing a component emulsified with the emulsifier.

### Means for Solving the Problems

The inventors have found that the suppression of the variation in particle diameter among the separated hydrophilic nanoparticles while avoiding the denaturation of the polymers can be achieved by, instead of cleaving all together the hydrogen bonds originating from granules including the conjugates of polycondensation polymer particles that include hydroxyl groups, firstly producing a relaxed product, in which a higher-order structure of conjugates has been relaxed, and subsequently cleaving the hydrogen bonds within the conjugates, to accomplish the present invention. Specifically, the present invention provides the following.
(1) In a first aspect of the present invention, a process for producing an emulsifier, comprising: a dispersion step of dispersing, into water, granules comprising conjugates of saccharide polymer particles that include hydroxyl groups, to thereby prepare a dispersion,
   a relaxed-product producing step of producing a relaxed product, wherein a higher-order structure of the conjugates has been relaxed, by swelling the granules and cleaving hydrogen bonds originating from the granules under reversible conditions that permit restoration of cleaved hydrogen bonds,
   a first restoring step of partially restoring the cleaved hydrogen bonds,
   a particle separation step of cleaving the hydrogen bonds within the conjugates, and separating the saccharide polymer particles into water,
   a step of diluting an emulsifier-producing material obtained after the first restoring step with water, before the particle separation step, and
   a second restoring step of partially restoring the cleaved hydrogen bonds,
   wherein cleavage of the hydrogen bonds under reversible conditions is cleavage under mild conditions which permit the restoration of once-cleaved hydrogen bonds that can be confirmed by the reversible transition of the viscosity as a function of the change of the temperature,
   wherein the cleavage is carried out by physical treatment by heating and agitation which is performed at 70 to 90 °C.
   the first and/or second partially restoring step is performed by leaving the particles under non-treatment conditions at ambient temperature, without agitation, and in the absence of any chemical agent for several hours.
(2) In a second aspect of the present invention, the relaxed-product producing step comprises increasing and decreasing the viscosity of the dispersion.
(3) In a third aspect of the present invention, the saccharide polymer is a naturally-occurring polymer.
(4) In a fourth aspect of the present invention, the first restoring step is performed under non-treatment conditions for several hours, wherein the restoring of the hydrogen bonds can be confirmed by the reversible transition of the viscosity as a function of the change of the temperature.
(5) In a fifth aspect of the present disclosure (not within the scope of the claims), a process for producing an emulsifier-producing material is provided, including: a dispersion step of dispersing, into water, granules including conjugates of polycondensation polymer particles that include hydroxyl groups, to thereby prepare a dispersion, and
   a relaxed-product producing step of producing a relaxed product, in which a higher-order structure of the conjugates has been relaxed, by swelling the granules and cleaving hydrogen bonds originating from the granules under reversible conditions.
(6) In a sixth aspect of the present disclosure (not within the scope of the claims), the process according to the fifth aspect is provided, in which the relaxed-product producing step includes increasing and decreasing the viscosity of the dispersion.
(7) In a seventh aspect of the present disclosure (not within the scope of the claims), the process according to the fifth or sixth aspect is provided, which further includes a first restoring step of partially restoring the cleaved hydrogen bonds.
(8) In an eight aspect of the present disclosure (not within the scope of the claims), the process according to any one of the fifth to seventh aspects is provided, in which the polycondensation polymer is a naturally-occurring polymer.
(9) In a ninth aspect of the present disclosure (not within the scope of the claims), the process according to the eight aspect is provided, in which the naturally-occurring polymer is a polysaccharide.
(10) In a tenth aspect of the present disclosure (not within the scope of the claims), a process for producing an emulsifier using the emulsifier-producing material produced by the process according to any one of the fifth to ninth aspects is provided, in which,
   the process including a particle separation step of cleaving the hydrogen bonds within the conjugates, and separating the polycondensation polymer particles into water.
(11) In an eleventh aspect of the present disclosure (not within the scope of the claims), the process according to the tenth aspect is provided, which further includes a step of diluting the emulsifier-producing material with water, before the particle separation step.
(12) In a twelfth aspect of the present disclosure (not within the scope of the claims), the process according to the tenth or eleventh aspect is provided, which further includes a second restoring step of partially restoring the cleaved hydrogen bonds.
(13) In a thirteenth aspect of the present disclosure (not within the scope of the claims), an emulsifier for an orally administered composition in use for the emulsification of an orally administered composition is provided, in which the emulsifier for an orally administered composition is produced by the process according to any one of the tenth to twelfth aspects, and the polycondensation polymer is a naturally-occurring polymer.
(14) In a fourteenth aspect of the present disclosure (not within the scope of the claims), an orally administered composition is provided, which contains the emulsifier according to the thirteenth aspect, and an emulsified product emulsified with the emulsifier.

### Effects of the Invention

According to the present invention, a relaxed product, in which the higher-order structure of the conjugates has been relaxed, is produced from the granules including conjugates of the polycondensation polymer particles that include hydroxyl groups, and subsequently the hydrogen bonds within the conjugates are cleaved, which allows the inhibition of the variation in particle diameter among the separated hydrophilic nanoparticles. In addition, any severe treatment that promotes the denaturation of the polycondensation polymer can be avoided, and therefore the impairment of the emulsifying functions of the hydrophilic nanoparticles can be inhibited.

### BRIEF DESCRIPTION OF THE DRAWINGS

FIG. 1 shows size distribution of the hydrophilic nanoparticles in the emulsifier produced by the process according to an embodiment of the present invention; and
FIG. 2 shows size distribution of the hydrophilic nanoparticles in the emulsifier produced by the process according to Comparative Example.

### PREFERRED MODE FOR CARRYING OUT THE INVENTION

Hereinafter, an embodiment of the present invention will be explained, and should by no means be construed as limiting the present invention.

### Process for Producing Emulsifier-Producing Material

The process for producing the emulsifier-producing material according to the present invention involves a dispersion step and a relaxed-product producing step. Details of the respective steps will be explained.

### Dispersion Step

In the dispersion step, the granules including the conjugates of the polycondensation polymer particles that include hydroxyl groups are dispersed into water, to prepare a dispersion. Since residual aggregates of the granules deteriorate the efficiency of subsequent steps such as swelling, the aggregates of the granules are eliminated, or the amount thereof is reduced, by dispersing the granules into water.

The polycondensation polymer that includes hydroxyl groups may be either a naturally-occurring polymer or a synthetic polymer, and may be selected as appropriate, depending on the use application of the emulsifier. Nonetheless, naturally-occurring polymers are preferred in terms of their superior safety and cheapness in general, and saccharide polymers described hereinbelow are more preferred in terms of their superior emulsifying function.

The saccharide polymers are polymers having glucoside structures, and exemplified by cellulose, starch, and the like. Examples of the saccharide polymers include: those produced by microorganisms using as their components a number of saccharides selected from monosaccharides such as ribose, xylose, rhamnose, fucose, glucose, mannose, glucuronic acid, and gluconic acid; naturally-occurring polymers such as xanthan gum, gum arabic, guar gum, karaya gum, carrageenan, pectin, fucoidan, quince seed gum, trant gum, locust bean gum, galactomannan, curdlan, gellan gum, fucogel, caseins, gelatin, starch, and collagen; semi-synthetic polymers such as methylcellulose, ethylcellulose, methyl hydroxypropyl cellulose, carboxymethylcellulose, hydroxymethylcellulose, hydroxypropylcellulose, sodium carboxymethylcellulose, alginic acid propylene glycol ester, cellulose crystals, starch-sodium acrylate graft polymers, hydrophobized hydroxypropyl methylcelluloses; synthetic polymers such as polyvinyl alcohol, polyvinyl pyrrolidone, carboxy vinyl polymers, polyacrylic acid salts, polyethylene oxide.

Dispersing operation may be carried out by a commonly known method, depending on the type of the polycondensation polymer(s) employed; for example, the starch and the like can disperse adequately in water at ambient temperature, whereas the xanthan gum and the like are routinely added into hot water.

The amount of the granules in the dispersion may be adjusted as appropriate in view of operability and demand for mass production. Specifically, an excessively large amount of the granules is prone to render the operation such as agitation difficult due to high viscosity of the granules after swelling, whereas an excessively small amount of the granules is disadvantageous from a mass production aspect. Accordingly, the amount of the granules may be adjusted as appropriate in view of these circumstances, depending on the type of the polycondensation polymer(s) employed, and is normally no greater than about 1% by mass.

### Relaxed-Product Producing Step

In the relaxed-product producing step, the relaxed product, in which the higher-order structure of the conjugates has been relaxed, is generated by swelling the granules and further cleaving the hydrogen bonds originating from the granules under reversible conditions. This leads to the relaxation of the higher-order structure, which gives rise to a situation for easy separation of the polycondensation polymer particles, while the emulsifying functions of the polycondensation polymer particles can be maintained by restoring the hydrogen bonds.

The swelling of the granules can lead to hydration of the polycondensation polymer particles, and result in the effect of the efficient cleavage of the hydrogen bonds, and the like. The swelling of the granules can be normally confirmed by clearness of the granules, increase in the viscosity of the dispersion, and the like. It is to be noted that the swelling may be carried out by a commonly known method depending on the polycondensation polymer employed.

The cleavage of the hydrogen bonds under reversible conditions is cleavage under mild conditions which permit the restoration of once-cleaved hydrogen bonds. In the conjugates, relative order of easy cleavage is expected to be as follows: hydrogen bonds participating in the formation of the higher-order structure > hydrogen bonds between the particles > bonds within the particles > bonds within the polycondensation polymers. In the present invention, since the hydrogen bonds are cleaved under mild conditions, the cleavage of the bonds within the polycondensation polymers can be avoided, and the emulsifying functions of the polycondensation polymer particles can be maintained. It is to be noted that the cleavage of the hydrogen bonds can be confirmed by the decrease in the viscosity of the dispersion, the observation of the relaxation of the higher-order structure of the conjugates with a microscope, and the like.

The cleavage of the hydrogen bonds can be carried out by physical treatments such as heating and agitation, and/or chemical treatments such as treatment with agents (for example, urea and thiourea). Heating temperature, agitation speed, the amount of the added agent(s), treatment time and the like are controlled so that the cleavage of the hydrogen bond is reversible. Although specific conditions may be set as appropriate, depending on the type of the polycondensation polymer(s) employed, in the case of the saccharide polymers, mild agitation at 70° C to 90°C, preferably about 80°C, over a period of 20 minutes to 40 minutes, preferably about 30 minutes, may be sufficient. It is to be noted that the reversible cleavage of the hydrogen bonds can be confirmed by the reversible transition of the viscosity as a function of the change of the temperature.

### First Restoring Step

In the present invention, the first restoring step of partially restoring the cleaved hydrogen bonds is preferably included. Through this step, the hydrogen bonds between the particles are restored, and cleavage distribution of the hydrogen bonds among the particles in a particle separation step (which will be described hereinbelow) is uniformized, and the variation in particle diameter among the resultant particles can be inhibited. In addition, since the hydrogen bonds within the particles are also restored, the impairment of the emulsifying property of the particles can be prevented.

The restoring in the first restoring step may be performed by leaving the particles under non-treatment conditions (for example, at ambient temperature, without agitation, and in the absence of any chemical agent) for several hours. The restoring of the hydrogen bonds can be confirmed by the reversible transition of the viscosity as a function of the change of the temperature. This step may be actively performed in the production lines, or passively performed during the storage and distribution of the emulsifier-producing material.

In the emulsifier-producing material thus produced, the variation in particle diameter among the hydrophilic nanoparticles separated in the step described below can be inhibited, and the impairment of the emulsifying functions of the hydrophilic nanoparticles can be also inhibited, owing to the relaxation of the higher-order structure of the conjugates as well as the restorability of the hydrogen bonds between the particles and within the particles.

### Process for Producing Emulsifier

The process for producing the emulsifier according to the present invention includes the particle separation step, and employs the emulsifier-producing material produced by the above-described process.

### Particle Separation Step

In the particle separation step, the hydrogen bonds within the conjugates contained in the emulsifier-producing material are cleaved, and the polycondensation polymer particles are separated into water. Due to the restoring of the hydrogen bonds between the particles, the distribution of the cleaved hydrogen bonds is substantially homogeneous, and, as a result, the variation in particle diameter among the separated polycondensation polymer particles are inhibited. It is to be noted that the separation of the polycondensation particles is not limited to the isolation of individual polycondensation particles, but encompasses the separation of the polycondensation particles as an agglomerate of the polycondensation particles.

The cleavage of the hydrogen bonds in this step is not particularly limited as long as the cleavage occurs under conditions in which the covalent bonds within the polycondensation polymers remain substantially uncleaved, and the cleavage may be typically carried out in the same manner as the cleavage of the hydrogen bonds in the aforementioned relaxed-product producing step. In other words, the cleavage of the hydrogen bonds can be carried out by physical treatments such as heating and agitation, and/or chemical treatments such as treatment with a certain agent (for example, urea and thiourea). The heating temperature, agitation speed, the amount of the added agents, treatment time and the like are controlled so that the covalent bonds within the polycondensation polymers are not cleaved. Although specific conditions may be set as appropriate, depending on the type of the polycondensation polymer(s) employed, in the case of the saccharide polymers, mild agitation at 70° C to 90°C, preferably about 80°C, over a period of 20 minutes to 40 minutes, preferably about 30 minutes, may be sufficient.

Such cleavage of the hydrogen bonds may be carried our until the hydrophilic nanoparticles (the generic term inclusive of one polycondensation polymer particle and more than one agglomerates) are obtained in a desired yield, in which the particle size of the hydrophilic nanoparticles as measured by the particle size analyzer FPAR (from Otsuka Electronics Co., Ltd.) is in the range of 50 nm to 800 nm. The hydrophilic nanoparticles having such a particle size have proven to exhibit superior emulsifying functions (for example, Japanese Unexamined Patent Application, Publication No. 2006-239666). Nonetheless, it should be noted that if the cleavage of the hydrogen bonds is carried out unduly, the bonds within the polycondensation polymer particles may be adversely affected.

### Dilution

It is preferred in view of the efficiency during its production, shipping and the like, for the emulsifier-producing material to be produced such that it contains a reasonably high concentration of the granules dispersed in water; however, higher concentration of the granules may reduce cleavage efficiency of the hydrogen bonds within the conjugates, due to the presence of the relaxed-product of the conjugates in high density. Therefore, it is preferred that the process for producing the emulsifier according to the present invention further includes the step of diluting the emulsifier-producing material with water, before the particle separation step. This can ensure the efficiency of the particle separation step, while ensuring the efficiency of the production, shipping and the like of the emulsifier-producing material. Nonetheless, in the case where the relaxed-product in the emulsifier-producing material employed is dilute, further dilution will not be greatly needed. Thus, the dilution factor may be adjusted as appropriate, depending on the density of the relaxed-product in the emulsifier-producing material employed.

### Second Restoring Step

It is preferred that the process for producing the emulsifier according to the present invention further includes the second restoring step of partially restoring the cleaved hydrogen bonds. Through this step, the hydrogen bonds within the hydrophilic nanoparticles can be restored and, hence the original emulsifying functions can be restored.

The restoring in the second restoring step is not particularly limited, and may be typically carried out in the same manner as the above-described first restoring step. In other words, the restoring in the second restoring step may be performed by leaving the particles under non-treatment conditions (for example, at ambient temperature, without agitation, and in the absence of any chemical agent) for several hours. The restoring of the hydrogen bonds can be confirmed by the reversible transition of the viscosity as a function of the change of the temperature. This step may be actively performed in the production lines, or passively performed during the storage and distribution of the emulsifier.

### Emulsifier

The emulsifier thus produced has reduced variation in particle diameter among the hydrophilic nanoparticles, in which the particle diameter is in the range of 50 nm to 800 nm, as measured by the particle size analyzer FPAR (from Otsuka Electronics Co., Ltd.), and further, the impairment of the emulsifying functions of the hydrophilic nanoparticles is inhibited due to the absence of any severe treatment that promotes the denaturation of the polycondensation polymers. Thus, the emulsifier as described above has homogeneous and superior emulsifying functions.

The emulsifier according to the present invention can be used for emulsifying various oily substances. Specifically, emulsions in which an oily phase containing an oily substance and an aqueous phase are dispersed can be produced by mixing the emulsifier and the oily substance, with or without the addition of a substance partitioned into the aqueous phase as appropriate. Herein, the oily substance refers to substances containing an oil alone or as a main component. Detailed procedure for the use of the emulsifier is described in Japanese Patent No. 3855203.

Moreover, emulsifiers obtained by using naturally-occurring polymers as the polycondensation polymer are preferably used for emulsifying substances utilized for living organisms, such as orally administered compositions (for example, beverages, food products, and orally administered formulations), external preparations, cosmetics, and agrochemicals, since the emulsifiers are more superior in safety. Thus, the present invention also provides an orally administered composition which contains an emulsifier for an orally administered composition obtained by using a naturally-occurring polymer as the polycondensation polymer and an emulsified product emulsified with the emulsifier for orally administered composition. The orally administered composition thus obtained is more superior in safety.

However, applications of the emulsifier according to the present invention are not limited to the above examples; the emulsifier according to the present invention allows for the formation of emulsified-dispersed systems exhibiting superior thermal stability and aging stability in an interface between a functional oily base and water, or between a functional granule and water, and the like. Thus, the emulsifying agent according to the present invention can be used for realizing stable emulsification over an extended period of time over a wide temperature range. In addition, irrespective of the required HLB values of the oleum to be emulsified or surface conditions of the functional granules, such oily substances can be emulsified and/or dispersed with the use of a single emulsifying agent, and hence the emulsifying and/or dispersing agent according to the present invention is applicable for emulsifying hydrocarbon-based oleums and silicon-based oleums. Thus, it is also applicable for emulsifying plural kinds of oils present in admixture all together. Therefore, the hydrophilic nanoparticles and emulsifier according to the present invention can be used for emulsifying water into various oils such as light oils, fuel oil A, fuel oil C, tar, biodiesel fuels, reclaimed oils, waste edible oils, cosmetic oils, edible oil, and oleums for industrial use (for example, silicon oils, kerosene).

### EXAMPLES

### Example

Starch (potato starch) granules were added into water such that the concentration thereof was 1% by mass, and dispersed by agitating the water, to prepare a dispersion. The dispersion was heated to 80°C with agitation, and the agitation was continued until the white starch granules became clear, to cause the granules to be swelled. During the swelling, the viscosity of the dispersion was increased.

Subsequently, the dispersion was kept agitated at 80°C for 30 minutes, and then left to settle at room temperature overnight. During the heating, the viscosity of the dispersion was decreased.

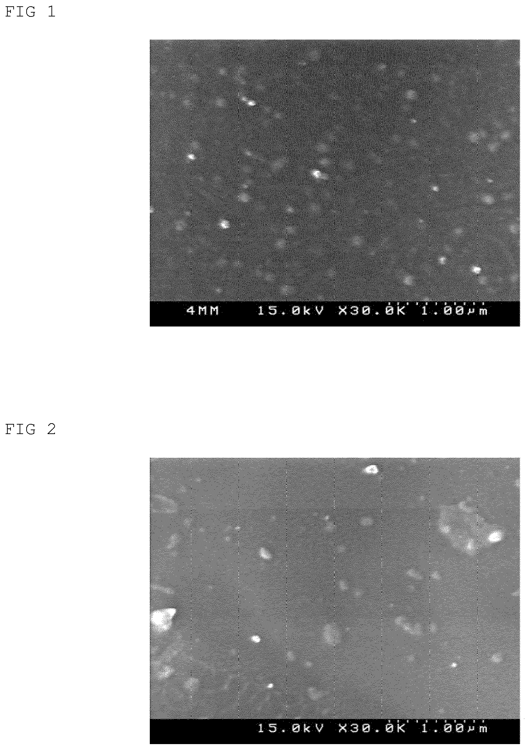

Then, the fluid after the settlement was diluted with water such that the ratio of the amount of the starch and water was set as shown in Table 1, and the diluted fluid was heated to 80°C with agitation, and was kept agitated at 80°C for 30 minutes. Subsequently, the fluid was allowed to cool to room temperature. A photograph taken during observation with a microscope of the substances in the fluid after cooling is shown in FIG. 1. FIG. 1 shows that in the fluid after settlement, the starch particles are discrete. Moreover, the size distribution of the fluid was measured using the particle size analyzer FPAR (from Otsuka Electronics Co., Ltd.), and a peak indicative of the presence of the hydrophilic nanoparticles was confirmed in the particle size range of 70 nm to 200 nm.

### Comparative Example

Starch (potato starch) granules and water were agitated according to the method disclosed in Japanese Unexamined Patent Application, Publication No. 2006-239666, such that the ratio of the amount of the starch and water was set as shown in Table 2, and the resultant dispersion was heated to 90°C, and then cooled to room temperature. A photograph taken during observation with a microscope of the substances in the fluid is shown in FIG. 2. It can be seen from FIG. 2 that the discrete starch particles of the fluid according to Comparative Example were irregular in size, and a considerable amount of conjugates of indiscrete particles are still present. Moreover, the size distribution of the particles in the fluid was measured using the particle size analyzer FPAR (from Otsuka Electronics Co., Ltd.), and a peak indicative of the presence of the hydrophilic nanoparticles was confirmed in the particle size range of 0.1 µm to 1 µm, indicating the particle size distribution of the hydrophilic nanoparticles according to Comparative Example is broader, compared to that for the hydrophilic nanoparticles according to Example.

### Evaluation

The respective emulsifiers obtained in Example and Comparative Example were agitated with liquid paraffin at room temperature in the ratio shown in Tables 1 and 2, and emulsified, and then the emulsification state of the resultant fluids was evaluated. The evaluation criteria are as follows.
A: no phase separation, B: separation resulting from the difference in specific gravity (coacervation), C: separation
1: O/W emulsion, 2: W/O emulsion, 3: W/O emulsion and separated aqueous phase

**[Table 1]**

| Examples | 1 | 2 | 3 | 4 | 5 | 6 | 7 | 8 | 9 | 10 | 11 |
|---|---|---|---|---|---|---|---|---|---|---|---|
| Starch | 0.18 | 0.16 | 0. 14 | 0.12 | 0.10 | 0. 08 | 0.07 | 0.06 | 0.05 | 0.04 | 0.02 |
| Water | 89.82 | 79.84 | 69. 86 | 59. 88 | 49.9 | 39.92 | 34. 93 | 29. 94 | 24. 95 | 19. 96 | 9. 98 |
| Liquid paraffin | 10 | 20 | 30 | 40 | 50 | 60 | 65 | 70 | 75 | 80 | 90 |
| Emulsification stability (one month/room temperature) | A | A | A | A | A | A | A | A | A | A | B |
| Emulsification state | 1 | 1 | 1 | 1 | 1 | 1 | 1 | 2 | 2 | 2 | 2 |

**[Table 2]**

| Comparative Examples | 1 | 2 | 3 | 4 | 5 | 6 | 7 | 8 | 9 | 10 | 11 |
|---|---|---|---|---|---|---|---|---|---|---|---|
| Starch | 0.18 | 0.16 | 0.14 | 0.12 | 0.10 | 0.08 | 0.07 | 0.06 | 0.05 | 0.04 | 0.02 |
| Water | 89.82 | 79.84 | 69.86 | 59.88 | 49.9 | 39.92 | 34.93 | 29.94 | 24.95 | 19.96 | 9.98 |
| Liquid paraffin | 10 | 20 | 30 | 40 | 50 | 60 | 65 | 70 | 75 | 80 | 90 |
| Emulsification stability (one month/room temperature) | B | B | B | B | B | B | A | A | A | B | C |
| Emulsification state | 1 | 1 | 1 | 1 | 1 | 1 | 2 | 2 | 2 | 2 | 3 |

As shown in Tables 1 and 2, phase separation was observed for all emulsions employing the emulsifier according to Comparative Example, whereas phase separation was not observed for all emulsions employing the emulsifier according to Example, except for the case of the use of 90% by mass of liquid paraffin. In addition, the emulsifier according to Comparative Example could not emulsify no less than 90% by mass of liquid paraffin, whereas the emulsifier according to Example produced emulsions in all the cases, including the case of 90% by mass of liquid paraffin.

## Claims

1. A process for producing an emulsifier, comprising: a dispersion step of dispersing, into water, granules comprising conjugates of saccharide polymer particles that include hydroxyl groups, to thereby prepare a dispersion,
a relaxed-product producing step of producing a relaxed product, wherein a higher-order structure of the conjugates has been relaxed, by swelling the granules and cleaving hydrogen bonds originating from the granules under reversible conditions that permit restoration of cleaved hydrogen bonds,
a first restoring step of partially restoring the cleaved hydrogen bonds,
a particle separation step of cleaving the hydrogen bonds within the conjugates, and separating the saccharide polymer particles into water,
a step of diluting an emulsifier-producing material obtained after the first restoring step with water, before the particle separation step, and
a second restoring step of partially restoring the cleaved hydrogen bonds,
wherein cleavage of the hydrogen bonds under reversible conditions is cleavage under mild conditions which permit the restoration of once-cleaved hydrogen bonds that can be confirmed by the reversible transition of the viscosity as a function of the change of the temperature,
wherein the cleavage is carried out by physical treatment by heating and agitation which is performed at 70 to 90 °C in 20 to 40 minutes,
the first and/or second partially restoring step is performed by leaving the particles under non-treatment conditions at ambient temperature, without agitation, and in the absence of any chemical agent for several hours.

2. The process according to claim 1, wherein the relaxed-product producing step comprises increasing and decreasing the viscosity of the dispersion.

3. The process according to any one of claim 1 or 2, wherein the saccharide polymer is a naturally-occurring polymer.

4. The process according to any one of claims 1 to 3, wherein the first restoring step is performed under non-treatment conditions for several hours, wherein the restoring of the hydrogen bonds can be confirmed by the reversible transition of the viscosity as a function of the change of the temperature.

## Patentansprüche

1. Ein Verfahren zur Herstellung eines Emulgators, umfassend:
einen Dispergierschritt zum Dispergieren von Granulaten, die Hydroxylgruppen enthaltende Konjugate von Saccharid-Polymer-Partikeln umfassen, in Wasser, um eine Dispersion herzustellen,
einen ein Relaxationsprodukt-herstellenden Schritt zur Herstellung eines relaxierten Produktes, in dem eine Struktur höherer Ordnung der Konjugate durch das Quellen der Granulate und das Spalten der in den Granulaten vorhandenen Wasserstoffbrückenbindungen unter reversiblen Bedingungen, die das Wiederherstellen von gespaltenen Wasserstoffbrückenbindungen erlauben, relaxiert wird,
einen ersten Wiederherstellungsschritt zum teilweisen Wiederherstellen von gespaltenen Wasserstoffbrückenbindungen,
einen Partikeltrennschritt, in dem die Wasserstoffbrückenbindungen in den Konjugaten gespalten werden und die Saccharid-Polymer-Partikel in Wasser abgeschieden werden,
vor dem Partikeltrennschritt einen Schritt zur Verdünnung eines Emulgator-erzeugenden Materials, das nach dem ersten Wiederherstellungsschritt erhalten wird, mit Wasser, und
einen zweiten Wiederherstellungsschritt zum partiellen Wiederherstellen von gespaltenen Wasserstoffbrückenbindungen,
wobei das Spalten der Wasserstoffbrückenbindungen unter reversiblen Bedingungen das Spalten unter milden Bedingungen darstellt, die die Wiederherstellung von einmal gespaltenen Wasserstoffbrückenbindungen erlauben, was durch den reversiblen Übergang der Viskosität als Funktion der Veränderung der Temperatur bestätigt werden kann,
wobei das Spalten durch eine physikalische Behandlung mittels Erhitzen und Rühren vorgenommen wird, das bei 70 bis 90°C für 20 bis 40 Minuten ausgeführt wird,
wobei der erste und/oder zweite partielle Wiederherstellungsschritt durchgeführt wird, indem die Partikel unter Nicht-Behandlungs-Bedingungen bei Raumtemperatur, ohne mechanisches Rühren und in Abwesenheit jeglicher chemischer Agentien für mehrere Stunden stehengelassen werden.

2. Das Verfahren gemäß Anspruch 1, wobei der Schritt zur Herstellung eines relaxierten Produktes das Erhöhen und das Verringern der Viskosität der Dispersion umfasst.

3. Das Verfahren gemäß einem der Ansprüche 1 oder 2, wobei das Saccharid-Polymer ein natürlich vorkommendes Polymer ist.

4. Das Verfahren gemäß einem der Ansprüche 1 bis 3, wobei der erste Wiederherstellungsschritt unter Nicht-Behandlungs-Bedingungen für mehrere Stunden durchgeführt wird, wobei die Wiederherstellung der Wasserstoffbrückenbindungen durch den reversiblen Übergang der Viskosität als eine Funktion der Änderung der Temperatur bestätigt werden kann.

## Revendications

1. Procédé de production d'un émulsifiant, comprenant :
une étape de dispersion consistant à disperser, dans de l'eau, des granules comprenant des conjugués de particules de polymère de saccharide qui comprennent des groupes hydroxyle, pour préparer ainsi une dispersion,
une étape de production de produit détendu consistant à produire un produit détendu, dans laquelle une structure d'ordre supérieur des conjugués a été détendue, par gonflement des granules et par clivage des liaisons hydrogène provenant des granules dans des conditions réversibles qui permettent la restauration des liaisons hydrogène clivées,
une première étape de restauration consistant à restaurer partiellement les liaisons hydrogène clivées,
une étape de séparation des particules consistant à cliver les liaisons hydrogène dans les conjugués et à séparer les particules de polymère de saccharide dans de l'eau,
une étape de dilution d'un matériau produisant un émulsifiant obtenu après la première étape de restauration avec de l'eau, avant l'étape de séparation des particules, et
une deuxième étape de restauration consistant à restaurer partiellement les liaisons hydrogène clivées,
dans lequel le clivage des liaisons hydrogène dans des conditions réversibles est un clivage dans des conditions douces qui permettent la restauration de liaisons hydrogène une fois clivées qui peut être confirmée par la transition réversible de la viscosité en fonction du changement de la température,
dans lequel le clivage est effectué par un traitement physique par chauffage et agitation qui est effectué à une température de 70 à 90°C en 20 à 40 minutes,
la première et/ou la deuxième étape de restauration partielle est effectuée en laissant les particules dans des conditions de non-traitement à température ambiante, sans agitation, et en l'absence de tout agent chimique pendant plusieurs heures.

2. Procédé selon la revendication 1, dans lequel l'étape de production de produit détendu comprend l'augmentation et la diminution de la viscosité de la dispersion.

3. Procédé selon l'une quelconque des revendications 1 ou 2, dans lequel le polymère de saccharide est un polymère naturel.

4. Procédé selon l'une quelconque des revendications 1 à 3, dans lequel la première étape de restauration est réalisée dans des conditions de non traitement pendant plusieurs heures, dans lequel la restauration des liaisons hydrogène peut être confirmée par la transition réversible de la viscosité en fonction du changement de la température.
